# EUROPÄISCHE PATENTSCHRIFT

(11) **EP 1 342 446 B1**
(45) Veröffentlichungstag und Bekanntmachung des Hinweises auf die Patenterteilung: **25.10.2006**
(21) Anmeldenummer: 03005058.7
(22) Anmeldetag: 06.03.2003
(51) Int. Cl.: A61B 1/005

(54) **Endoskopschaft mit beweglichem Endabschnitt**
Endoscope with a flexible distal tip
Endoscope avec une extrémité distale flexible

(30) Priorität: 07.03.2002 DE 10209986
(43) Veröffentlichungstag der Anmeldung: 10.09.2003
(73) Patentinhaber: STM Medizintechnik Starnberg GmbH, 69469 Weinheim (DE)
(72) Erfinder: Pauker, Fritz, 86316 Friedberg (DE); Viebach, Thomas, Weberstrasse 17 69469 Weinheim (DE)
(74) Vertreter: TBK-Patent

(56) Entgegenhaltungen:
- EP-A- 0 488 322
- WO-A-01/80935
- DE-A- 4 435 644
- US-A- 4 890 602
- US-A- 4 976 191
- US-A- 5 662 587

## Beschreibung

Die vorliegende Erfindung betrifft einen Endoskopschaft gemäß dem Oberbegriff des Patentanspruchs 1.

Endoskope sind Geräte, insbesondere zur Exploration von Hohlräumen oder röhrenartigen Kanälen des Körpers, insbesondere für medizinische Zwecke. Besonders eingeführt haben sich Endoskope zur Exploration der Speiseröhre, des Magens, des Zwölffingerdarms vom Magen aus, des Darms vom Anus aus, der Harnröhre, der Blase und der Harnleiter. Ein derartiges Endoskop ist an seinem vorderen Ende mit einer Beleuchtungseinrichtung sowie mit einer Optik zur visuellen Erfassung des davor liegenden Bereichs des zur explorierenden Körperhohlraums oder Körperkanals ausgerüstet. Während bis vor kurzem die vor dem vorderen Ende des Endoskops erfasste, optische Information normalerweise mittels einer Faseroptik durch das Endoskop nach hinten zu seinem Bedienungsabschnitt übertragen worden ist, stellt jetzt der Einbau eines Kamerachips an der vorderen Endoskopspitze sowie die elektrische Bildübertragung und die Darstellung der gewonnenen optischen Information auf einem Bildschirmmonitor den neuesten Stand der Technik dar.

Endoskope weisen ferner in der Regel einen sog. Arbeitskanal auf, durch den diverse Arbeitsinstrumente eingeführt und bedient werden können. Beispielsweise werden kleine Zangen zur Entnahmen von Gewebeproben, Biopsienadeln, beheizbare Schneiddrähte, kleine Scheren, Koagulationselektroden oder dergleichen eingeführt, um ggf. operative Maßnahmen an beschädigtem Gewebe durchzuführen. Schließlich sind in der Regel ein Fluidkanal für Spülflüssigkeit und Bedienungsdrähte zum Abwinkeln des vorderen Endes des Endoskops in mehrere Richtungen vorhanden. Diese Bedienungsdrähte werden wiederum durch einzelne Kanäle innerhalb des Endoskopschafts zu dessen vorderem bzw. distalem Ende geführt, um dieses bis zu 160° in Gegenrichtung des Endoskopschafts dreidimensional zu biegen.

Hierbei ergeben sich nunmehr wesentliche Probleme insbesondere bezüglich des taktilen Gefühls, welches auf den Bediener während des Biegevorgangs des distalen Endes vermittelt wird. Es besteht daher die Gefahr, dass beispielsweise bei der Exploration des Darms, die Darmwand während des Biegevorgangs des distalen Endes beschädigt wird. Darüber hinaus muss eine exakte Positionierbarkeit des distalen Endes an der zu explorierenden und ggf. zu behandelnden Stelle möglich sein, was eine ausreichende Biegefähigkeit bei gleichzeitiger Steifigkeit nach Erreichen der vorbestimmten Biegeposition erforderlich macht.

Zur Lösung dieses Problems sieht ein Stand der Technik gemäß der DE 100 10 932 A1 vor, den distalen Endabschnitt aus einer Mehrzahl von längs neben- bzw. übereinander geschichteter balgförmiger Scheibenkörper bzw. Schwellkörper zu bilden, von denen jeweils zwei diamterisch zueinanderliegend eine Körperschicht ausbilden und von denen zwei längs, unmittelbar benachbarte Körperpaare 90° zueinander phasenverschoben sind. Hierdurch ergibt sich eine Konstruktion, in welcher die einzelnen scheibenartigen Schwellkörper in Längsrichtung gesehen abwechselnd auf null und sechs Uhr gemäß der einen Schicht und auf drei und neun Uhr gemäß der benachbarten, bzw. darüber angeordneten Schicht zu liegen kommen. Auf diese Weise ist es möglich, durch entsprechende Betätigung der in der gleichen Winkelposition sich befindlichen Balge bei deren Aufblähen oder Zusammenziehen eine Abkrümmung des distalen Endes in die beabsichtigte Richtung zu erzielen, wobei mit Erreichen der vorbestimmten Abwinklungsposition des distalen Endes die Schwellkörper in dieser Stellung quasi eingefroren werden und damit die Haltung des distalen Endes fixiert wird.

Versuche haben indessen gezeigt, dass, die Herstellung eines distalen Endes aus den vorstehend beschriebenen scheibenförmigen Schwellkörpern überaus teuer ist, da diese hydraulisch und fluiddicht miteinander verbunden werden müssen. Die Fertigung der Schwellkörper selbst ist damit äußerst aufwendig und die Montage der Schwellkörper arbeitsintensiv. Für einen Einweg-Artikel ist daher der so hergestellte Endoskopschaft aus Kostengründen eher ungeeignet.

Dokument WO/80935 A1 offenbart einen Endoskopshaft entsprechend dem Oberbegriff des Patentanspruchs 1.

Angesichts dieser Problematik ist es Aufgabe der Erfindung, eine Konstruktion für einen distalen Endabschnitt eines Endoskopschafts bereitzustellen, die preisgünstig herstellbar ist und trotzdem eine verbesserte Funktionalität hat.

Diese Aufgabe wird erfindungsgemäß durch einen Endoskopschaft mit den Merkmalen des Patentanspruchs 1 gelöst.

Demzufolge hat der erfindungsgemäße Endoskopschaft einen beweglichen distalen Endabschnitt, der mittels einer Betätigungsvorrichtung abkrümmbar ist. Der distale Endsbschnitt hat dabei zumindest ein bezüglich der Längsachse des Endoskopschafts dezentral angeordnetes, sich längs des distalen Endabschnitts erstreckendes Schlauchelement, das aus einer Mehrzahl von unmittelbar, d.g. auf einer Geraden übereinander angeordneten Faltenbälgen einstückig gebildet ist und dabei einen durchgehenden Druckraum ausbildet. Bei einer Druckbeaufschlagung dieses Druckraums dehnt sich das Schlauchelement in dessen Längsrichtung aus und bewirkt so aufgrund seiner dezentralen Anordnung zwangsläufig eine Abkrümmung des distalen Endabschnitts. Jedes Schlauchelement bildet in seinem Querschnitt ein Kreissegment, wobei jeweils zueinander benachbarte Schlauchelemente an ihren Flanken aneinander anliegen.

Die Anordnung des vorstehend beschriebenen schlauchartigen Faltenbalgs hat gegenüber dem eingangs genannten Stand der Technik den Vorteil, dass er in einfacher Weise, beispielsweise durch Strangpressen bereits als ein einziges Bauteil herstellbar ist und als solches auch nicht mehr beim Einbau in den distalen Endabschnitt erst zusammengesteckt und verschweißt werden muß.

Auf Grund der Längserstreckung des schlauchförmigen Faltenbalgelements kann unter Umständen bei Druckbeaufschlagung nicht nur eine Längsausdehnung sondern auch eine mehr oder weniger große Aufweitung in Radialrichtung eintreten, die ggf. zu einer radialen Ausdehnung des gesamten distalen Endabschnitts führen könnte. Um dies sicher zu vermeiden ist es als eine vorteilhafte Weiterbildung des Erfindungsgegenstands vorgesehen, ein Mittel zur Reduzierung einer Aufblähung des jeweiligen Schlauchelements quer zu dessen Längsrichtung anzuordnen. Gemäß Anspruch 3 ist das Mittel zur Reduzierung einer Aufblähung ein an einer Außenseite des Schlauchelements angeordnetes Stützkorsett. Der Vorteil dieser Ausgestaltung besteht darin, dass die Anordnung des äußeren Stützkorsetts als optionale Maßnahme in Abhängigkeit der zu erwartenden Druckverhältnisses sowie des vorhandenen Einbauraums vorgenommen werden kann und auch unterschiedlich dimensionierte Korsette verwendet werden können. Dies ermöglicht die Ausbildung eines quasi Einheits-Schlauchelements, der dann für die einzelnen Verwendungsfälle geeignet gemacht werden kann. Die bewirkt eine weitere Verringerung des Gesamtherstellungskosten.

Weitere vorteilhafte Ausgestaltungen der Erfindung sind Gegenstand der übrigen Unteransprüche.

Die Erfindung wird nachstehend anhand bevorzugter Ausführungsbeispiele unter Bezugnahme auf die begleitenden Zeichnungen näher erläutert.
Fig. 1 zeigt den vorderen Abschnitt eines Endoskopschafts samt beweglichem distalen Ende gemäß einem ersten bevorzugten Ausführungsbeispiel der Erfindung,
Fig. 2A zeigt das bewegliche distale Ende gemäß der Fig. 1 in Vergrößerung,
Fig. 2B zeigt ein bewegliches distales Ende gemäß einem zweiten bevorzugten Ausführungsbeispiel der Erfindung,
Fig. 2C zeigt ein bewegliches distales Ende gemäß einem dritten bevorzugten Ausführungsbeispiel der Erfindung,
Fig. 3A und 3B zeigen einen Schaftkopf in Seiten- und Draufsicht mit dem beweglichen Endabschnitt gemäß dem ersten bevorzugten Ausführungsbeispiel, wobei der Schaftkopf natürlich auch auf den beweglichen Endabschnitt gemäß dem zweiten oder dritten Ausführungsbeispiel aufsetzbar ist,
Fig. 4A bis 4C zeigen das Bewegungsverhalten des erfindungsgemäßen distalen Endes bei Druckbeaufschlagung und
Fig. 5 zeigt eine Betätigungsvorrichtung des erfindungsgemäßen distalen Endes.

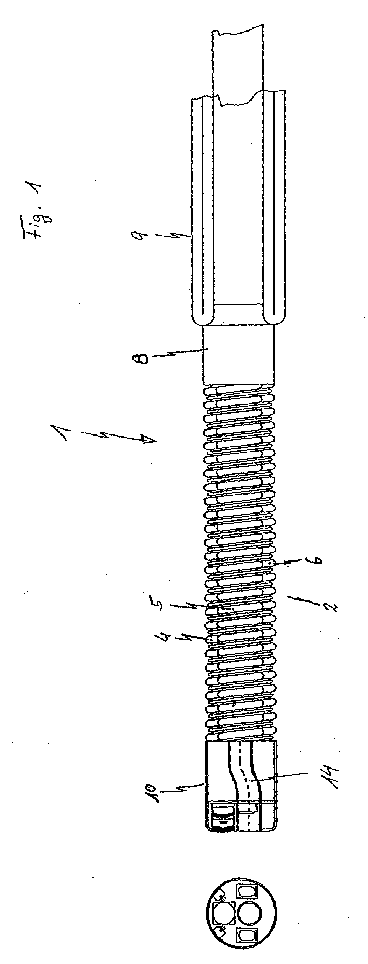

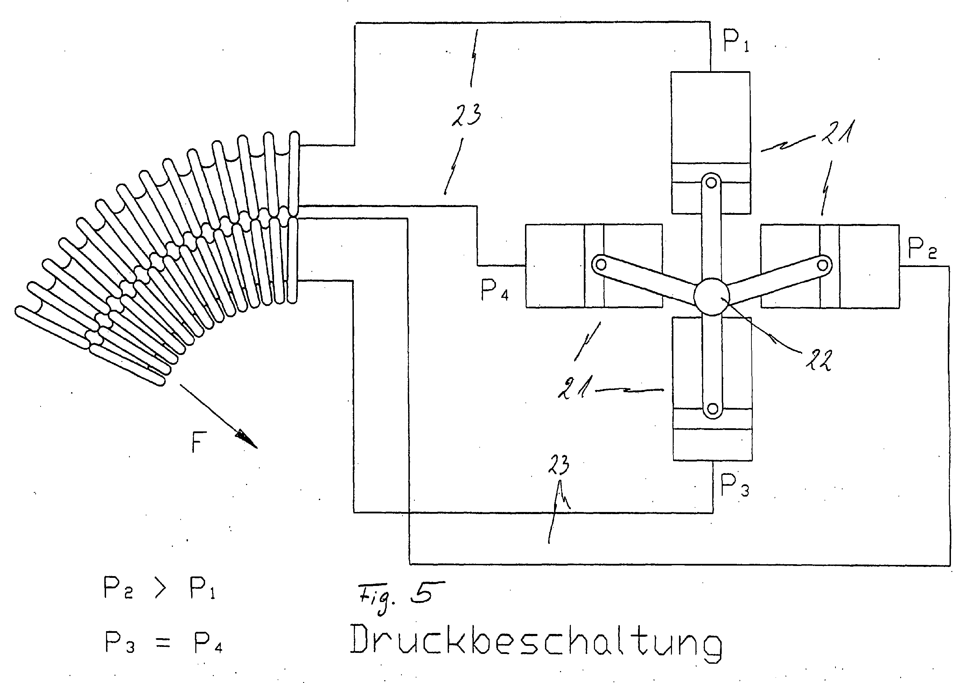

Wie insbesondere aus der Fig. 1 zu entnehmen ist, hat der Endoskopschaft 1 gemäß dem ersten bevorzugten Ausführungsbeispiel der Erfindung einen beweglichen distalen Endabschnitt 2, der mittels einer Betätigungsvorrichtung 3 abkrümmbar ist, wie sie nachfolgend noch anhand der Figur 5 als eine mögliche, jedoch auch anders gestaltbare Ausführungsform beschrieben wird. Der distale Endabschnitt 2 hat dabei zumindest ein (vorliegend vier) dezentral angeordnetes, sich längs des distalen Endabschnitts erstreckendes Schlauchelement 4-7, das zur Erreichung dieser Längserstreckung aus einer Mehrzahl (mindestens zwei) von, im wesentlichen auf einer Geraden übereinander angeordneten Faltenbälgen einstückig gebildet ist. Dieses schlauchartig langgestreckte Faltenbalgelement bildet einen einzigen, d.h. integrierten Druckraum aus, derart, dass sich das Faltenbalgelement bei Druckbeaufschlagung im wesentlichen nur in Längsrichtung ausdehnt, wodurch sich der bewegliche Endabschnitt infolge der dezentralen Anordnung des zumindest einen Faltenbalgelement abkrümmt.

Der Endoskopschaft 1 hat des weiteren einen hinteren Schaftabschnitt 8, an dessen hinteres, in der Fig. 1 nicht mehr dargestelltes Ende die Betätigungsvorrichtung 3 für den distalen Endabschnitt 2 angeschlossen ist und der vorzugsweise in einer Vorschubeinrichtung 9 bestehend aus einem aus dem Stand der Technik bekannten Stülpschlauchsystem eingesetzt ist. Der hintere Schaftabschnitt 8 beinhaltet eine Anzahl von voneinander getrennten Arbeits-, Versorgungs- und Betätigungskanälen, durch die beispielsweise Hydraulikfluid zur individuellen Druckbeaufschlagung der Schlauchelemente 4-7, Reinigungsflüssigkeiten, Arbeitsgerätschaften oder einfach Leitungskabel für optische oder elektrische Einrichtungen an einem am distalen Ende des Endabschnitts 2 angeordneten Schaftkopf 10 geführt werden können.

Am vorderen Ende des hinteren Schaftabschnitts 8 ist der bewegliche distale Endabschnitt 2 fest angeschlossen, derart, dass die im hinteren Schaftabschnitt 8 ausgebildeten Kanäle in entsprechende Kanäle innerhalb des distalen Endabschnitts 2 fluiddicht münden. Vorzugsweise ist der distale Endabschnitt 2 hierfür unlösbar mit dem hinteren Schafabschnitt 8 verschweißt, wobei natürlich auch eine lösbare Steck- oder Schraubverbindung denkbar ist.

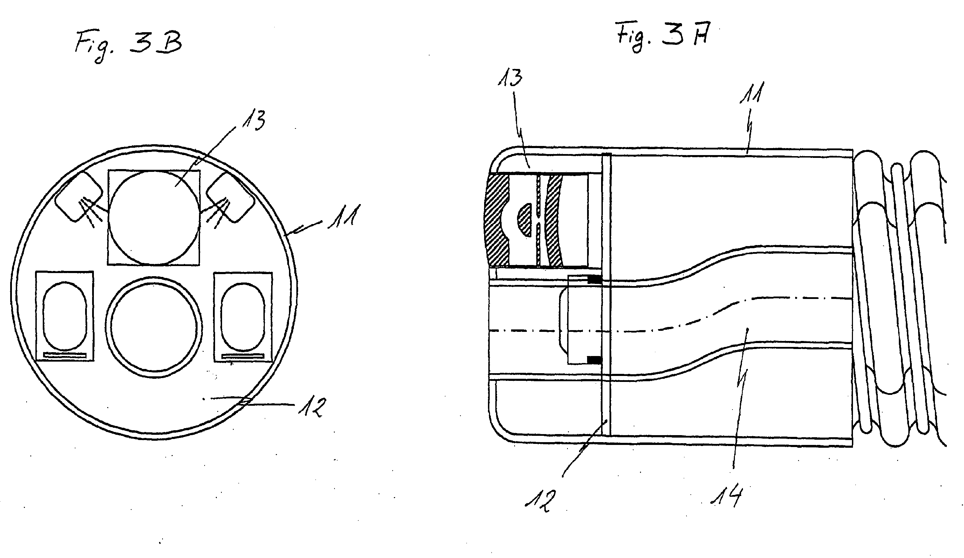

Am vordersten Ende des distalen Endabschnitts 2 ist der Schaftkopf 10 angeschlossen, vornehmlich auf die gleiche Weise, wie der distale Endabschnitt 2 am hinteren Schaftabschnitt 8. Wie in der Fig. 1 andeutungsweise und in den Fig. 3A und 3B in Vergrößerung dargestellt ist, besteht der Schaftkopf 10 aus einem zumindest an der vorderen Stirnseite geschlossenen Gehäuse 11, in dem eine im Parallelabstand zur Gehäusestirnseite ausgerichtete Platine 12 eingesetzt ist. An der Platine 12 ist eine Photozelle oder ein Kamerachip 13, sowie Beleuchtungsköper montiert, die jeweils durch entsprechende Öffnungen an der Gehäusestirnseite dichtend herausragen. Des weiteren ist in dem Gehäuse 11 ein zentraler Arbeitskanal 14 verlegt, der ebenfalls durch die Platine 12 hindurchgeführt ist und in eine Öffnung an der Gehäusestirnseite mündet. Wie aus der Draufsicht gemäß der Fig. 3B zu entnehmen ist, sind beidseits des Arbeitskanals 14 weitere Versorgungskanäle, beispielsweise zur Zufuhr von Reinigungsflüssigkeit oder Luft vorgesehen.

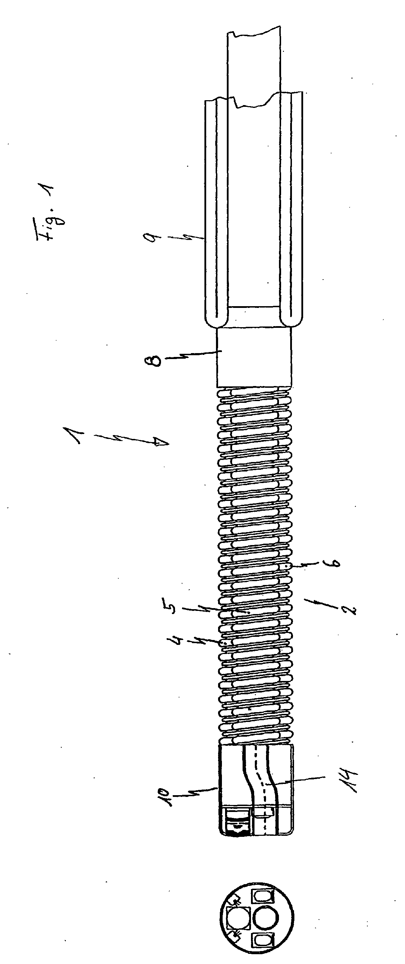

In den Fig. 2A bis 2C sind verschiedene Ausführungsformen des distalen, abkrümmbaren Endabschnitts 2 sowohl in Seiten- wie auch in Querschnittsansicht dargestellt.

Demzufolge hat der bewegliche distale Endabschnitt 2 gemäß der Fig. 2A vier Schlauchelementen 4, 5, 6, 7, die jeweils im Querschnitt zu einem Viertelkreis ausgebildet sind. Jedes Schlauchelement 4-7 besteht aus einem schlauchartigen Hohlkörper aus einem möglichst undehnbaren jedoch flexiblen Material wie einem armierten Kunststoff, dessen Wandstärke im äußeren Teilkreisabschnitt stärker ist als an den beiden geraden Flankenabschnitten. Des weiteren ist jedes Schlauchelement 4-7 in der Querschnittsansicht zu einem inneren Teilkreis ausgebildet, der beim Zusammenbau aller vier Schlauchelemente 4-7 einen zentralen Innenkanal 15 umschließt, der mit dem entsprechenden Arbeitskanal 14 im Schaftkopf 10 sowie dem Arbeitskanal im hinteren Schaftabschnitt 8 verbunden ist.

In der Seitenansicht gemäß der Fig. 2A ist die Faltstruktur jedes Schlauchelements 4-7 zu erkennen. Demnach hat jedes Schlauchelement 4-7 eine Vielzahl von radial vorstehenden Ausstülpungen 16 oder Falten, die bezüglich der Längsachse des Schlauchelements 4-7 schräg ausgerichtet sind. Hierdurch ergibt sich nach Zusammenbau aller vier Schlauchelemente 4-7 durch die Ausstülpungen 16 eine äußere, geschlossene Wendelform.

Des weiteren umgibt den distalen Endabschnitt 2 eine Art äußeres Stützkorsett, welches ein radiales Aufblähen des jeweiligen Schlauchelements 4-7 bei Druckbeaufschlagung mit verhindern soll. Im vorliegenden Ausführungsbeispiel gemäß der Fig. 2A ist dieses äußere Stützkorsett aus einer Spiralfeder 17 ausgebildet, deren Steigung und Abmessung der äußeren Wendelform des distalen Endabschnitts 2 angepasst ist und die zwischen die radial vorstehenden Ausstülpungen 16, d.h. in die Täler der Wendelform eingesetzt ist. Vorzugsweise ist der Spiralfederdraht so gestaltet (beispielsweise rechteckig oder oval), dass er ein möglichst kraftloses Abkrümmen der Feder 17 erlaubt, jedoch eine große Widerstandskraft gegen ein radiales Aufweiten der Feder 17 entwickelt. Die Feder 17 übt ferner eine einwärts gerichtete Radialkraft auf die Schlauchelemente 4-7 aus, um diese in ihrer Relativlage zu halten.

In der Fig. 2B ist ein zweites bevorzugtes Ausführungsbeispiel für den beweglichen, distalen Endabschnitt 2 gemäß der Erfindung dargestellt, wobei für zum ersten Ausführungsbeispiel gleiche Bauteile gleiche Bezugszeichen verwendet sind.

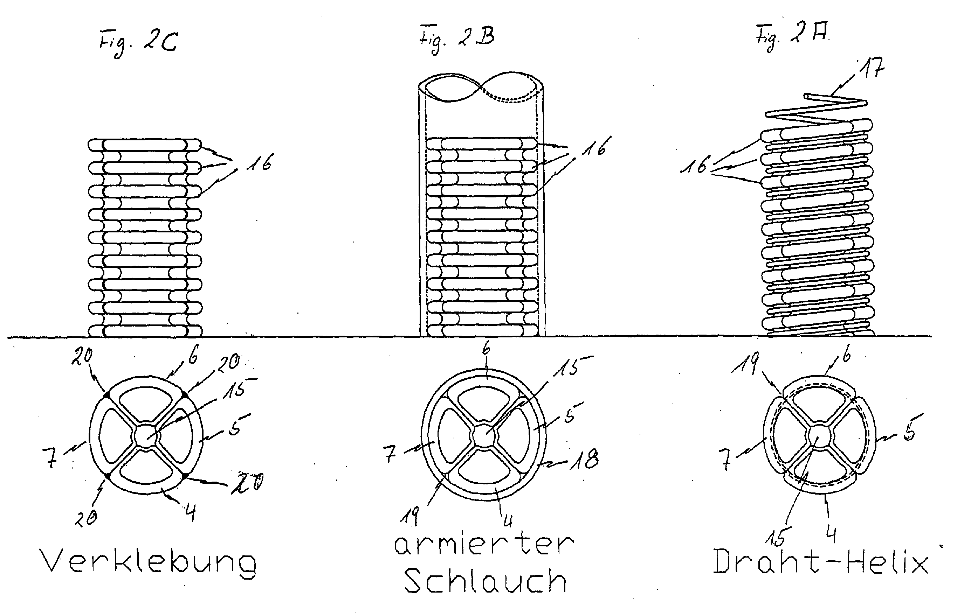

In diesem zweiten Ausführungsbeispiel ist das Stützkorsett aus einer elastischen, schlauchartigen Hülle 18 ausgebildet, die unter einer bestimmten Vorspannung über die zusammengebauten Schlauchelemente 4-7 gezogen ist, um hierdurch eine radial nach innen gerichtete Kraft auf die Schlauchelemente 4-7 auszuüben. Um eine noch festere Verbindung zwischen der Hülle 18 und den Schlauchelementen 4-7 zu erhalten, kann die Hülle 18 auf die Schlauchelemente 4-7 aufgeschrumpft oder mit diesen verklebt sein. Wie ferner aus der Fig. 2 zu ersehen ist, ist die Faltenbalgstruktur jedes Schlauchsegments 4-7 gemäß dem zweiten Ausführungsbeispiel unterschiedlich zu jener des ersten Ausführungsbeispiels. Da die Spiralfeder 17 durch die Schlauchhülle 18 ersetzt wird, ist eine wendelförmige Faltenbalgstruktur gemäß der Fig. 2A zwar in diesem Fall zwar möglich aber nicht erforderlich. Vielmehr sind die radial nach außen ausgerichteten Ausstülpungen 16 jedes Schlauchelements 4-7 als radiale, parallelbeabstandete Ringsegmente ausgebildet, die sich im montierten Zustand der vier Schlauchelemente 4-7 zu umlaufenden, geschlossenen Ringen ergänzen. Zusätzlich oder anstelle der elastischen Hülle 18 können einzelne Metall- oder Kunststoffreifen (nicht weiter dargestellt) in die Täler zwischen den Ausstülpungen 16 eingelegt sein, die eine radiale Aufweitung der Schlauchelemente 4-7 bei Druckbeaufschlagung mit verhindern.

Schließlich ist in der Fig. 2C ein drittes bevorzugtes Ausführungsbeispiel für einen distalen Endabschnitt gemäß der Erfindung dargestellt, wobei auch hier gleiche Bezugszeichen für bereits vorstehend beschriebene Bauteile vergeben sind.

In diesem Fall sind die Schlauchelemente 4-7 wie im zweiten Ausführungsbeispiel ausgebildet, können aber auch wie im ersten Ausführungsbeispiel, d.h. mit wendelförmig ausgerichteten Ausstülpungen 16 ausgebildet sein. Im zusammengebauten Zustand bilden sich aufgrund fertigungstechnisch bedingter Kantenrundungen an den Viertelkreissegmentförmigen Schlauchelementen 4-7 längs sich erstreckende, durchgehende Einkerbungen 19. Diese Einkerbungen 19 können als Aufnahme eines Stützkorsetts gemäß der Erfindung ausgenutzt werden. Im vorliegenden Fall sind die längsverlaufenden Einkerbungen 19 mit einer Klebmasse 20 aufgefüllt, die nach deren Abbinden zum Einen ein außenseitiges Verkleben der jeweils aneinanderliegenden Schlauchelemente 4-7 bewirken und zum Anderen als Verstärkungsstränge dienen. Diese Form eines Stützkorsetts kann bereits mit dem Zusammenbau der Schlauchsegmente 4-7 ausgebildet werden, ohne dass sich die Abmessungen des distalen Endabschnitts 2 in radialer Richtung vergrößern. Insofern eignen sich diese Verklebungen nicht nur als alleinige Maßnahme zur Reduzierung radialer Aufweitungen infolge Druckbeaufschlagung, sondern insbesondere als Ergänzung jener technischer Maßnahmen, wie sie anhand der Fig. 2A und 2B bereits beschrieben worden sind.

In der Fig. 5 ist beispielhaft die Betätigungseinrichtung für die Schlauchelemente 4-7 des distalen Endabschnitts 2 dargestellt. Diese hat demzufolge vier Kolben/Zylinder- oder alternativ hierzu zwei Doppelkolben/Zylinderanordnungen 21, die mittels eines Betätigungsgestänges 22 miteinander verbunden sind. Jeder Zylinder, oder im Fall der zweiseitig (Doppelhub) wirkenden Doppelkolbenzylinder jede Zylinderseite ist mit einem der Schlauchsegmente 4-7 hydraulisch über die Betätigungskanäle innerhalb des Endoskopschafts verbunden, derart, dass bei einer Betätigung eines Zylinders (Zylinderseite) zur Druckbeaufschlagung eines Schlauchelements gleichzeitig ein anderer Zylinder (Zylinderseite) zur entsprechenden Druckverminderung des jeweils diametrisch gegenüberliegenden Schlauchelements betätigt wird. Auf diese Weise wird zumindest ein Schlauchelement in Längsrichtung gedehnt, während das diametrisch gegenüberliegende Schlauchelement in Längsrichtung verkürzt wird, wodurch eine Abkrümmung des distalen Endabschnitts 2 in Richtung des sich verkürzenden Schlauchelements bewirkt wird.

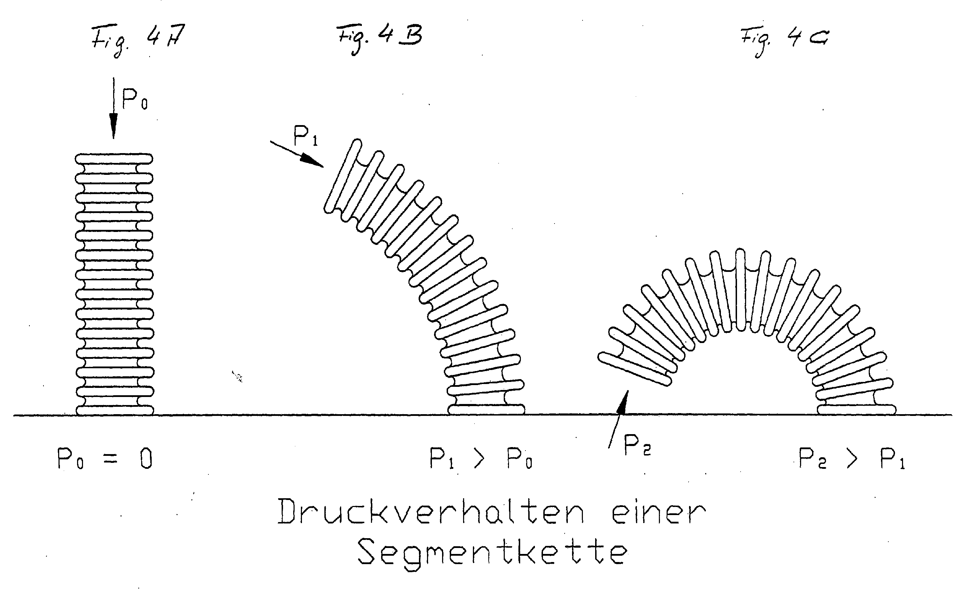

Bevor die Funktionsweise des erfindungsgemäßen Endoskopschafts insbesondere des distalen Endabschnitts 2 anhand der Fig. 4A bis 4C und 5 beschrieben wird, sei noch auch die folgenden möglichen Abwandlungen zu den vorstehend beschriebenen Ausführungsbeispielen hingewiesen:

Je nach Einsatz- und Verwendungszweck des Endoskops kann dieses manuell innerhalb eines zu untersuchenden Hohlraums im Ganzen gedreht werden. In diesem Fall ist es also nicht erforderlich, den beweglichen, distalen Endabschnitt in sämtliche Richtungen abkrümmbar zu gestalten. Vielmehr genügt es dann, eine Abkümmung in nur eine Richtung (zweidimensional) zu ermöglichen. Dies lässt sich bereit mit nur einem oder zwei der dezentral angeordneten Schlauchelemente erreichen, die in diesem Fall auch nicht viertelkreisförmig sein brauchen sondern eine beliebig andere Querschnittsform erhalten können. Durch diese Maßnahme reduziert sich nicht nur der Konstruktionsaufwand des gesamten Endoskopschafts, sondern es wird auch zusätzlich Hohlraum für weitere oder größere Arbeits- und Versorgungskanäle bereitgestellt.

Der in der Fig. 2B dargestellte hüllenförmige Schlauch 18 kann mit einer Armierung versehen sein, derart, dass diese eine radiale Aufweitung der Hülle 18 verhindert, die Elastizität des Hüllenmaterials in Längsrichtung jedoch weitgehend unbeeinflusst lässt.

Obgleich die vorstehend beschriebenen Schlauchelemente einen im wesentlichen geraden, längs sich erstreckenden Hohlraum ausbilden ist es auch möglich, die Schlauchelemente spiralförmig auszugestalten, um bei einer Druckbeaufschlagung mit im wesentlichen hieraus resultierender Längenausdehnung eine Art progressive Kreisbewegung des Schaftkopfs 10 zu bewirken.

All diese Abwandlungen sowie die vorstehend beschriebenen Ausführungsbeispiele haben jedoch die Ausbildung des schlauchförmig langgestreckten Faltenbalgelements mit einem einzigen, integralen Hohlraum gemeinsam, das als ein einziges, integrales Bauteil in einem einzigen Fertigungsschritt einfach und preisgünstig herstellbar und montierbar ist. Des weiteren dient sowohl die Wandstärkenverteilung sowie insbesondere das äußere Stützkorsett dazu eine Radialaufweitung möglichst zu verhindern und nur eine Längsausdehnung bei Druckbeaufschlagung zuzulassen.

Zur Beschreibung der Funktionsweise wird auf die Fig. 4A bis 4C und 5 verwiesen.

Wird demzufolge das Betätigungsgestänge 22 manuell verschoben, werden die daran angeschlossenen Kolben/Zylindereinheiten 21 entsprechend betätigt, wodurch Hydraulikfluid über Hydraulikleitungen 23 und die Hydraulikkanäle innerhalb des Endoskopschafts 1 zu den Schlauchelementen 4-7 geleitet oder von diesen abgeführt wird. Durch den hierbei entstehenden Druckaufbau in zumindest einem der Schlauchelemente 4-7 wird dieses in Längsrichtung ausgedehnt, wohingegen das diametrisch gegenüberliegende Schlauchelement durch ein Abziehen des Hydraulikfluids in Längsrichtung verkürzt wird. Wie vorstehend bereits angedeutet ist, verhindert oder reduziert das erfindungsgemäße äußere Stützkorsett ein radiales Aufblähen der Schlauchelemente 4-7, sodass der Volumenzustrom an Hydraulikfluid im wesentlichen nur in eine Längenausdehnung des entsprechenden Schlauchelements umgesetzt wird. Diese Wirkung des äußeren Stützkorsetts wird noch dadurch unterstützt, dass die radial äußere Wand jedes Schlauchsegments 4-7 gegenüber den Flankenseiten stärker ausgebildet ist und daher einen erhöhten Widerstand gegen Verformung, d.h. radiale Aufweitung bietet.

Durch die Längenunterschiede der angesteuerten Schlauchelemente wird der distale Endabschnitt, in welchem die Schlauchelemente verankert sind in die Richtung der kürzeren Schlauchelements abgekrümmt, wodurch sich der Schaftkopf 10 um bis zu 180° in jede Richtung bewegt werden kann. Auf diese Weise lässt sich Schaftkopf 10 in jede beliebige Position bewegen und dort durch Einspannen der jeweils vorherrschenden Hydraulikfluiddrücke quasi arretieren.
Bei der vorliegenden Erfindung handelt es sich um einen Endoskopschaft mit einem beweglichen distalen Endabschnitt (2), der mittels einer Betätigungsvorrichtung (3) abkrümmbar ist, wobei der distale Endsbschnitt (2) zumindest ein dezentral angeordnetes, sich längs des distalen Endabschnitts (2) erstreckendes Schlauchelement (4-7) hat, das aus einer Mehrzahl von unmittelbar übereinander angeordneten Faltenbälgen einstückig gebildet ist und dabei einen durchgehenden Druckraum ausbildet, um sich bei Druckbeaufschlagung in Längsrichtung auszudehen, wobei jedes Schlauchelement in seinem Querschnitt ein Kreissegment bildet, wobei jeweils zueinander benachbarte Schlauchelemente an ihren Flanken aneinander anliegen.

## Patentansprüche

1. Endoskopschaft mit einem beweglichen distalen Endabschnitt (2), der mittels einer Betätigungsvorrichtung (3) abkrümmbar ist und der eine Anzahl dezentral angeordneter, sich längs des distalen Endabschnitts (2) erstreckender Schlauchelemente (4-7) jeweils in Form eines Harmonikabalgs hat, welche somit jeweils einen durchgehenden Druckraum ausbilden, um sich bei individueller Druckbeaufschlagung in Längsrichtung auszudehen **dadurch gekennzeichnet, dass**
jeder Harmonikabalg (4-7) in seinem Querschnitt ein Kreissegment bildet, wobei jeweils zueinander benachbarte Harmonikabalge an ihren Flanken aneinander anliegen.

2. Endoskopschaft nach Anspruch 1, **gekennzeichnet durch** ein Mittel zur Reduzierung einer Aufblähung des jeweiligen Schlauchelements quer zu dessen Längsrichtung.

3. Endoskopschaft nach Anspruch 2, **dadurch gekennzeichnet, dass** das Mittel zur Reduzierung einer Aufblähung ein an einer Außenseite des Schlauchelements angeordnetes Stützkorsett ist.

4. Endoskopschaft nach Anspruch 3, **dadurch gekennzeichnet, dass** das Stützkorsett eine Spiralfeder ist.

5. Endoskopschaft nach Anspruch 3, **dadurch gekennzeichnet, dass** das Stützkorsett eine vorzugsweise armierte Schlauchhülle ist.

6. Endoskopschaft nach Anspruch 3, **dadurch gekennzeichnet, dass** das Stützkorsett durch eine Anzahl von längsbeabstandeten Reifen oder Ringen gebildet ist.

7. Endoskopschaft nach einem der vorstehenden Ansprüche, **dadurch gekennzeichnet, dass**
das Schlauchelement in seinem Querschnitt einen Viertelkreis bildet, wobei vier der kreissegmentförmigen Schlauchelemente an ihren Flanken aneinander anliegen.

8. Endoskopschaft nach Anspruch 7 in Verbindung mit einem der Ansprüche 3 bis 6, **dadurch gekennzeichnet, dass**
das Stützkorsett an der von den Schlauchelementen gemeinsam gebildeten radialen Außenseite angeordnet ist.

9. Endoskopschaft nach Anspruch 8, **dadurch gekennzeichnet, dass** das Stützkorsett eine zwei unmittelbar aneinander liegende Schlauchelemente verbindende, vorzugsweise längsverlaufende Klebewulst ist.

10. Endoskopschaft nach einem der vorstehenden Ansprüche, **dadurch gekennzeichnet, dass**
das Schlauchelement aus einem armierten Kunststoffmaterial besteht.

## Claims

1. An endoscope shaft having a movable distal end portion (2), which may be bent by means of an actuating device (3) and which has a number of non-centrally arranged hose elements (4-7) extending along the distal end portion (2) each in the form of accordion bellows, which thus in each case form a continuous pressure chamber, in order to expand longitudinally when exposed individually to pressure, **characterised in that** each accordion bellows (4-7) forms in cross-section a segment of a circle, wherein in each case adjacent accordion bellows adjoin one another at their side faces.

2. An endoscope shaft according to claim 1, **characterised by** a means for reducing distension of the respective hose element transversely of the longitudinal direction thereof.

3. An endoscope shaft according to claim 2, **characterised in that** the means for reducing distension is a supporting corset arranged on an outer side of the hose element.

4. An endoscope shaft according to claim 3, **characterised in that** the supporting corset is a coil spring.

5. An endoscope shaft according to claim 3, **characterised in that** the supporting corset is a preferably reinforced hose jacket.

6. An endoscope shaft according to claim 3, **characterised in that** the supporting corset takes the form of a number of longitudinally spaced hoops or rings.

7. An endoscope shaft according to any one of the preceding claims, **characterised in that** the hose element forms in cross-section a quarter circle, wherein four of the hose elements in the form of segments of circles adjoin one another at their side faces.

8. An endoscope shaft according to claim 7 in conjunction with any one of claims 3 to 6, **characterised in that** the supporting corset is arranged on the radial outer side formed jointly by the hose elements.

9. An endoscope shaft according to claim 8, **characterised in that** the supporting corset is a preferably longitudinally extending adhesive bead connecting two directly adjacent hose elements.

10. An endoscope shaft according to any one of the preceding claims, **characterised in that** the hose element consists of a reinforced plastics material.

## Revendications

1. Tige d'endoscope avec une section d'extrémité (2) distale flexible, qui peut se plier au moyen d'un dispositif d'actionnement (3) et qui présente une pluralité d'éléments tubulaires (4-7) disposés de manière décentrée, qui s'étendent le long de la section d'extrémité (2) distale, chacun en forme d'un soufflet d'accordéon, lesquels formant à chaque fois ainsi une chambre de pression sur toute la longueur pour pouvoir s'étendre en direction longitudinale lors de la pressurisation individuelle **caractérisée en ce que**
chaque soufflet en accordéon (4-7) forme dans sa section transversale un segment circulaire, chacun des soufflets en accordéon adjacents les uns aux autres s'appliquent les uns contre les autres sur leurs flancs.

2. Tige d'endoscope selon la revendication 1, **caractérisée par** un moyen pour réduire un gonflement de chacun des éléments tubulaires en diagonale par rapport à leur direction longitudinale.

3. Tige d'endoscope selon la revendication 2, **caractérisée en ce que** le moyen pour réduire un gonflement est un corset de protection disposé sur la face externe de l'élément tubulaire.

4. Tige d'endoscope selon la revendication 3, **caractérisée en ce que** le corset de protection est un ressort en spirale.

5. Tige d'endoscope selon la revendication 3, **caractérisée en ce que** le corset de protection est de préférence une enveloppe tubulaire armée.

6. Tige d'endoscope selon la revendication 3, **caractérisée en ce que** le corset de protection est formé par une pluralité d'anneaux ou de bagues espacé(e)s de manière longitudinale.

7. Tige d'endoscope selon l'une quelconque revendications précédentes, **caractérisée en ce que**
l'élément tubulaire forme dans sa section transversale un quart de cercle, quatre parmi les éléments tubulaires en forme de segment circulaire s'appliquant les contre les autres sur leurs flancs.

8. Tige d'endoscope selon la revendication 7 en liaison avec l'une quelconque des revendications 3 à 6, **caractérisée en ce que**
le corset de protection est disposé sur la face externe radiale formée en commun avec les éléments tubulaires.

9. Tige d'endoscope selon la revendication 8, **caractérisée en ce que** le corset de protection est un renflement s'étendant de préférence de manière longitudinale reliant deux éléments tubulaires appliqués directement l'un contre l'autre.

10. Tige d'endoscope selon l'une quelconque des revendications précédentes, **caractérisé en ce que**
l'élément tubulaire est fabriqué à partir d'un matériau plastique armé.
